(19) Europäisches Patentamt

European Patent Office

Office européen des brevets

(11) **EP 1 178 313 A2**

(12) **EUROPEAN PATENT APPLICATION**

(43) Date of publication:
06.02.2002 Bulletin 2002/06

(51) Int Cl.[7]: **G01N 33/20**

(21) Application number: 01306529.7

(22) Date of filing: 31.07.2001

(84) Designated Contracting States:
**AT BE CH CY DE DK ES FI FR GB GR IE IT LI LU MC NL PT SE TR**
Designated Extension States:
**AL LT LV MK RO SI**

(30) Priority: 31.07.2000 JP 2000232531

(71) Applicant: **National Institute for Materials Science**
**Tsukuba-shi, Ibaraki 305-0047 (JP)**

(72) Inventors:
• **Sawai, Tatsuaki, c/o Nat.Inst.for Mat.Science**
**Tsukuba-shi, Ibaraki (JP)**
• **Matsuoka, Sabura, c/o Nat.Inst.for Mat.Science**
**Tsukuba-shi, Ibaraki (JP)**

• **Abe, Takayuki, c/o Nat.Inst.for Mat.Science**
**Tsukuba-shi, Ibaraki (JP)**
• **Takeuchi, Etsuo, c/o Nat.Inst.for Mat.Science**
**Tsukuba-shi, Ibaraki (JP)**
• **Miyahara, Kensuke, c/o Nat.Inst.for Mat.Science**
**Tsukuba-shi, Ibaraki (JP)**
• **Hirukawa, Hisashi, c/o Nat.Inst.for Mat.Science**
**Tsukuba-shi, Ibaraki (JP)**
• **Tsuzaki, Kaneaki, c/o Nat.Inst.for Mat.Science**
**Tsukuba-shi, Ibaraki (JP)**
• **Kimura, Yuji, c/o Nat.Inst.for Mat.Science**
**Tsukuba-shi, Ibaraki (JP)**

(74) Representative: **Calamita, Roberto**
**Frank B. Dehn & Co., European Patent Attorneys,**
**179 Queen Victoria Street**
**London EC4V 4EL (GB)**

(54) **Method of estimating the fatigue strength of high tensile strength steel and method of producing a high fatigue strength material**

(57) The present invention provides a method of designing a high fatigue strength material in high tensile strength steel, comprising the steps of: obtaining the values of tensile strength $\sigma_B$ (unit thereof is MPa) and Vickers hardness Hv of the steel; measuring the flaw area of inclusion, when the fracture origin is located only at the surface of the steel; and estimating, in designing the high fatigue strength material, that the fatigue limit $\sigma_w$ (unit thereof is MPa) of the steel satisfies either $\sigma_W \geq 0.5\sigma_B$ or $\sigma_W \geq 1.6$Hv, when a square root of the flaw area, (area)$^{1/2}$ (unit thereof is m), contained in the steel is no larger than $45.8/\sigma_B^2$ or $4.47/Hv^2$. According to the present invention, a method of evaluating high fatigue strength material in high tensile strength steel, in which method the relationship between the flaw dimension (area) of ODA and the fatigue strength is considered, as well as a high fatigue strength material, can be provided.

**Description**

The present invention relates to a method of evaluating high fatigue strength material in high tensile strength steel and creation of a high fatigue strength material produced by the aforementioned evaluation method. More specifically, the present invention relates to a method of evaluating high fatigue strength material in high tensile strength steel and creation of a high fatigue strength material, which are useful for material designing and production of high tensile strength steel having gigacycle-level fatigue strength, which high tensile strength steel having gigacycle-level fatigue strength is advantageously used for reducing the weight and size and improving the performance of a mechanical structure or vehicle parts.

High tensile strength steel having excellently high fatigue strength of gigacycle level has been on demand, in order to reduce weight and size and enhance performances of mechanical structures and vehicle parts. However, in the case of high tensile strength steel of 1200-MPa-exceeding grade, the fatigue strength of the high tensile strength steel is rather decreased at the gigacycle range due to the internal fracture, that is, the fish eye-type fracture initiated by inclusions or structure cracking, that is, internal facet. Therefore, it is not technologically easy to produce high tensile strength steel having high fatigue strength. Further, a method of designing a material for producing high tensile strength steel having higher fatigue strength is hardly known, because the mechanism of the internal fracture as described above is quite complicated.

Examples of the method of designing a material for producing high tensile strength steel include a method which comprises the steps of: analyzing the size (area) and/or properties of flaws of the inclusion; measuring flaw area of the inclusion; and calculating the fatigue limit from the correlation equation which defines the relationship between the flaw area of the inclusion and the fatigue limit.

However, as this method calculates only an approximate fatigue limit value by simplifying the complicated mechanism of the internal fracture, the method is by no means a designing method for producing high tensile strength steel having a higher fatigue strength.

Although the situation is as described above, a new attempt has been made in recent years, as one method of solving the complicated mechanism of the internal fracture, in which attempt the optically dark area (ODA) which is presumably the fracture developed by hydrogen around the inclusion is observed and the relationship between the ODA (flaw area) and the fatigue strength is analyzed. If the formation mechanism of ODA (flaw area) is made clear in future, the discovery may contribute to providing a method of designing high tensile strength steel having excellent fatigue strength. However, the discovery has not been fully made yet and there does not exist a clear prospect of specifically designing such a method.

The invention of the present application has been achieved in consideration of the aforementioned problems of the prior art. The present invention has an object of providing a method of evaluating high fatigue strength material in high tensile strength steel and a high fatigue strength material itself, in which method the relationship between the flaw dimension (area) of ODA and the fatigue strength is considered.

In order to solve the aforementioned problems, the present invention proposes, as the first aspect, a method of evaluating a high fatigue strength material in high tensile strength steel, comprising the steps of: obtaining the values of tensile strength $\sigma_B$ (the unit thereof is MPa) and Vickers hardness Hv of the steel; and estimating, in designing the high fatigue strength material, that the fatigue strength $\sigma_w$ (the unit thereof is MPa) of the steel satisfies either $\sigma_w \geq 0.5\sigma_B$ or $\sigma_w \geq 1.6$Hv, when the fracture origin is located only at a surface of the steel and a square root of the flaw area, (area)$^{1/2}$ (the unit thereof is m), contained in the steel is no larger than $45.8/\sigma_B^2$ or $4.47/\text{Hv}^2$.

The present invention proposes, as the second aspect, a method of evaluating a high fatigue strength material in high tensile strength steel, comprising the steps of: obtaining the values of tensile strength $\sigma_B$ (the unit thereof is MPa) and Vickers hardness Hv of the steel; measuring the flaw area of inclusion, when the fracture origin is located inside the steel; and estimating, in designing the high'fatigue strength material, that the fatigue limit $\sigma_w$ (the unit thereof is MPa) of the steel satisfies a condition that $\sigma_w \geq 3.38$ (area$_i$)$^{-1/4}$.

The present invention proposes, as the third aspect, a method of evaluating a high tensile strength structure, which method enabling the evaluation of the high fatigue strength material according to the aforementioned first or second aspects, which method comprising the steps of: measuring the maximum inhomogeneous structure flaw area, (area$_{max,m}$), when the structure has been made homogeneous (i.e., the size of the inhomogeneous structure has been made small) or the structure has been made minute (i.e., the block width thereof has been reduced); setting the distribution of the maximum-minimum range of the maximum inhomogeneous structure flaw area, (area$_{max, m}$)$^{1/2}$, (the unit thereof is μm) within the range defined by the line: (area$_{max, m}$)$^{1/2} = 0$ and the line: (area$_{max, m}$)$^{1/2} = 0.9403y + 4.571$ (y is a standardizing parameter, the test standard area $S_0 = 6.2 \times 10^{-9}\text{m}^2$); and setting the distribution of the maximum-minimum range of the maximum block width d$_{max}$ (the unit thereof is μm) within the range defined by the line: d$_{max} = 0$ and the line: d$_{max} = 0.217y + 0.701$ (y is a standardizing parameter, the test standard area being $1 \times 10^{-10}\text{m}^2$) ;

The present invention proposes, as the fourth aspect, a method of producing a high fatigue strength material,

# EP 1 178 313 A2

which method comprising producing steel according to the method of evaluating a high fatigue strength material in high tensile strength steel of the aforementioned first and second aspects and the method of evaluating a high tensile strength structure of the aforementioned third aspect.

The present invention proposes, as the fifth aspect, a method of producing a high fatigue strength material, which method comprising producing steel according to the method of the aforementioned first aspect, by subjecting steel to a heating treatment for tempering the steel in high vacuum of $2 \times 10^{-6}$ Pa or higher.

Further, the present invention proposes, as the sixth aspect, a high fatigue strength material produced according to the method of evaluating a high fatigue strength material in high tensile strength steel of the aforementioned first and second aspects and the method of evaluating a high tensile strength structure of the aforementioned third aspect.

Fig. 1 is a diagram which shows the relationship between the static strength and the at $10^9$ cydes fatigue limit in an embodiment of the present invention.

Figs. 2(a)-(c) are scanning electron microscope (SEM) photographs of the internal fracture origin and the vicinities thereof.

Fig. 3 is a diagram which shows the relationship between the optically dark area (ODA) and the flaw area of inclusion, $(area)^{1/2}$ in the embodiment of the present invention.

Fig. 4 is a schematic diagram which illustrates a state of the internal fracture.

Fig. 5 is a diagram which shows the relationship between the flaw area, $(area)^{1/2}$, and the fatigue limit in the embodiment of the present invention.

Fig. 6 is a diagram which illustrates the process of the heating treatment.

Figs. 7(a)-(b) are optical microscope photographs of the inhomogeneous structure.

Fig. 8 is a diagram which statistically shows the maximum- minimum range of the inhomogeneous structure flaw area of inclusion in the embodiment of the present invention.

Figs. 9(a)-(b) are AFM images of blocks of a tempered martensite structure.

Fig. 10 is a diagram which statistically shows the maximum- minimum range of the maximum block width in the embodiment of the present invention.

Fig. 11 is a diagram which shows the result of the fatigue test of an ausform (AF) material (size of inclusions were under threshold) in the embodiment of the present invention.

Fig. 12 is a diagram which shows the result of the fatigue test of an AF material (size of inclusions were over threshold) in the embodiment of the present invention.

The invention of the present application has the features as described above. An embodiment of the present invention will be described hereinafter.

The present invention is significantly characteristic in that it proposes a method comprising the steps of: analyzing the correlation between the fatigue strength and the flaw area of inclusion (including the optically dark area (ODA) flaw dimension); and determining the flaw area, normally for the following three cases of: a) the case in which the fracture origin is located only at the surface; b) the case in which the fracture origin is located inside the steel; and c) the case in which the structure has been made homogeneous or the grain has been made minute; and thereby calculating the fatigue limit.

Accordingly, in the present invention, as the correlation between the fatigue strength and the area of inclusion is made obvious, the inclusion of desired size can be reliably provided, whereby high tensile strength steel having the same static strength but a higher fatigue strength, as compared with the conventional high tensile strength steel, can be produced and designing fatigue strength of high tensile strength steel can be more reliably carried out.

In the present invention, the flaw area of inclusion represents the square root of the flaw area in a cross section, which surface is normal to the load applying direction, of the inclusion, and has a length unit in terms of the dimension. The flaw area of the maximum inhomogeneous structure represents the square root of the maximum value of the flaw area in a cross section, which surface is normal to the load applying direction, of the inhomogeneous structure of the inclusion, and has a length unit in terms of the dimension.

Further, the present invention enables providing high tensile strength steel, which is produced by the aforementioned method of designing a high fatigue strength material. Specifically, the present invention provides high tensile strength steel having the fatigue strength as defined below.

$$\sigma_w \geq 0.5\ \sigma_B \text{ or } \sigma_w \geq 1.6^{HV},$$

wherein $\sigma_B$ is tensile strength (the unit thereof is MPa), Hv is Vickers hardness, and $\sigma_w$ is fatigue limit (the unit thereof is MPa).

It should be noted that high tensile strength steel having such excellent fatigue strength as described above has not been available up to now.

First, in steel having known tensile strength $\sigma_B$ (MPa) and Vickers hardness Hv, and specifically, in steel in which the fracture origin is located only at the surface thereof (in other words, in steel in which the surface fracture occurs prior to the internal fracture) (the aforementioned case **a)**), the method of obtaining the fatigue limit $\sigma_w$ will be described.

In this case, as the fracture origin is located only at the surface of the steel, it is assumed that the steel is high tensile strength steel which does not experience generation of optically dark area (ODA) in which the fatigue limit of the surface fracture, $\sigma_{wsurface}$, exceeds 0.5 $\sigma_B$ or 1.6 Hv. The maximum value of the flaw area in which surface fracture occurs prior to the internal fracture (the maximum flaw), $(area)^{1/2}_{max}$ (the unit thereof is m) can be expressed as follows, as a specific case of (the square root of) the flaw area (the unit thereof is m), by using the fatigue limit $\sigma_{w,surface}$ of the surface fracture.

Equation (1)

$$\frac{\left(\sqrt{area}\right)_{\max}}{\sqrt{\pi}} = \frac{1}{\pi}\left(\frac{\Delta K_{th}}{0.666\sigma_w, surface}\right)^2 \tag{1}$$

Here, $\Delta K_{th}$ is the lower limit value of the stress, that is, fatigue threshold, spreading coefficient range in which cracking proceeds. The general value thereof is 3 MPam$^{1/2}$, approximately. Accordingly, the maximum flaw area, $(area)^{1/2}_{max}$, is expressed as follows, by using tensile strength $\sigma_B$ (the unit thereof is MPa) and Vickers hardness Hv.

Equation (2)

$$(\sqrt{area})_{max} = 45.8/\sigma_B{}^2 \ or \ (\sqrt{area})_{max} = 4.47/Hv^2 \tag{2}$$

From the aforementioned equation (2), it is understood that, when the fracture origin is located only at the surface of steel and if the flaw dimension area, $(area)^{1/2}$, is no larger than $45.8/\sigma_B{}^2$ or $4.47/Hv^2$, the fatigue limit $\sigma_w$ of the steel satisfies either $\sigma_w \geq 0.5\sigma_B$ or $\sigma_w \geq 1.6Hv^2$. In other words, by designing a high fatigue strength material such that the material satisfies the aforementioned conditions, high tensile strength steel whose fatigue limit $\sigma_w$ satisfies either $\sigma_w \geq 0.5\sigma_B$ or $\sigma_w \geq 1.6Hv^2$ can be produced.

Next, in steel in which the fracture origin is located inside the steel (the aforementioned case **b)**), the method of obtaining the fatigue limit $\sigma_w$ will be described. When the flaw area of inclusion is expressed as $area_i$ (the unit of $(area_i)^{1/2}$ is m), since the fatigue limit at the internal fracture origin is preferentially dealt with, the fatigue limit $\sigma_w$ is expressed as follows.

Equation (3)

$$\sigma_w = \frac{\Delta K_{th}}{0.666\cdot\sqrt{\sqrt{\pi}\cdot}\sqrt{area_i}} \tag{3}$$

Here, when $\Delta K_{th}$ is 3 Mpa$\square$m$^{1/2}$, the $\sigma_w$ is expressed as follows.

Equation (4)

$$\sigma_w = 3.38(area_i)^{-1/4} \qquad \square 4 \square$$

That is, it is understood that, in the steel in which ODA is not generated and the inclusion area is not under control, the fatigue limit $\sigma_w$ of the steel has a value of $3.38\ (area_i)^{-1/4}$ or larger ($area_i$ is the measured flaw area of inclusion). In other words, by designing a high fatigue strength material such that the material satisfies the aforementioned conditions, high tensile strength steel whose fatigue limit $\sigma_w$ satisfies $\sigma_w \geq 3.38\ (area_i)^{-1/4}$ can be obtained.

Next, the aforementioned case **c)** in which the structure has been made homogeneous (i.e., the size of the inhomogeneous structure has been made small) or the structure has been made minute (i.e., the block width thereof has been reduced) will be described as the method of evaluating high tensile strength structure, which method enables designing a high fatigue strength material as described in the aforementioned **a)** and **b)**.

Specifically, in the case of **c)**, when the maximum inhomogeneous structure flaw area, $area_{max,m}$, is measured, the distribution of the maximum-minimum range of the maximum inhomogeneous structure flaw area, $(area_{max,\ m})^{1/2}$ is to be within the range defined by the line: $(area_{max,\ m})^{1/2} = 0$ and the line: $(area_{max,\ m})^{1/2} = 0.9403y + 4.571$, and the distribution of the maximum-minimum range of the maximum block width $d_{max}$ is to be within the range defined by the line: $d_{max} = 0$ and the line: $d_{max} = 0.217y + 0.701$. Here, the test standard area $S_0$ for obtaining the maximum inhomogeneous structure flaw area, $(area_m)^{1/2}$, is preferably $6.2 \times 10^{-9}$ m² or so, and the test standard area for obtaining the maximum block width $d_{max}$ is preferably $1 \times 10^{-10}$ m² or so.

The present invention will be described further in detail hereinafter, by the following non-limiting examples.

EXAMPLES

Example 1

The preparation of samples and pre-treatment

In order to demonstrate the precision of the method of evaluating a high fatigue strength material of the present invention, an example was actually carried out by using spring steel SUP12 and valve spring steel SWOSC-V. First, samples of these types of steel were prepared and pre-treated. Thereafter, the mechanical properties of the samples were examined.

The chemical components of spring steel SUP12 and valve spring steel SWOSC-V are shown in Table 1. Six different heats of samples including four different heats of spring steel SUP12 (heat A, heat B2, heat C1, heat D1) and two different heats of valve spring steel SWOSC-V (heat E2, heat F) were prepared, according to the types of heat. The shape, flaw area, conditions of the heating treatment, tensile strength $\sigma_B$ and Vickers hardness Hv of the steel samples are shown in Table 2. For the heat D1 of spring steel SUP12, two types of samples which differ from each other in the conditions of the heating treatment i.e., a normally-quenched-and-tempered material (a QT material) and an modified ausform material (an AF material) were prepared.

Table 1

| Types of Steel | Heat | Chemical Component (mass %) | | | | | | | | |
|---|---|---|---|---|---|---|---|---|---|---|
| | | C | Si | Mn | P | S | Cu | Ni | Cr | Al |
| SUP12 | A | 0.53 | 1.49 | 0.69 | 0.011 | 0.007 | 0.01 | 0.02 | 0.74 | 0.039 |
| | B2 | 0.56 | 1.50 | 0.67 | 0.014 | 0.008 | 0.01 | 0.01 | 0.72 | 0.021 |
| | C1 | 0.56 | 1.48 | 0.72 | 0.012 | 0.009 | - | - | 0.71 | 0.001 |
| | D1 | 0.57 | 1.48 | 0.72 | 0.008 | 0.007 | - | - | 0.73 | 0.005 |
| SWOSC-V | E2 | 0.57 | 1.46 | 0.63 | 0.008 | 0.006 | 0.01 | 0.01 | 0.68 | 0.001 |
| | F | 0.58 | 1.44 | 0.70 | 0.013 | 0.006 | 0.01 | 0.02 | 0.70 | 0.001 |

Table 2

| Shape of base material, Conditions of heating treatment, Tensile strength and Vickers hardness | | | | | | |
|---|---|---|---|---|---|---|
| Type of Steel | Heat | Shape and Area | Conditions of heating treatment | Tensile strength (MPa) | Vickers hardness HV (196N) | Note |
| SUP12 | A | Round Rod $\phi$ 21mm | Quenching at 845 °C $\times$ 30 minutes, oil cooling Tempering at 430 °C $\times$ 60 minutes, water cooling | 1720 | 516 | QT material |
| | B2 | Billet: 160 $\times$ 160 mm | | - | 530 | |
| | C1 | Round Rod $\phi$ 20mm | | - | 511 | |
| | D1 | Round Rod $\phi$ 20mm | | 1742 | 518 | |
| SWOSC-V | E2 | Billet: 160 $\times$ 160 mm | | - | 532 | |
| | F | Wire material $\phi$ 11mm | | - | 528 | |
| SUP12 | D1 | Round Rod $\phi$ 50mm | Modified ausforming at 845 °C $\times$ 30 minutes, air cooling, 800 °C, 50%, water cooling Tempering at 430 °C $\times$ 60 minutes, water cooling | 1770 | 534 | AF material |

First, for each type of the totally six types of steel described above, a graph of the maximum-minimum range was statistically prepared for obtaining the maximum flaw area, $(area_{max, m})^{1/2}$. In the present example, the flaw area of the maximum inclusion can be reliably employed when the fracture does not occur inside the steel but is located only at the surface of the steel.

By using the statistic graph of the maximum- minimum range, the maximum flaw area, $(area_{max, m})^{1/2}$, was obtained at the test standard area $S_0$ value of 0.482 mm$^2$. This procedure of obtaining $(area_{max, m})^{1/2}$ was repeated twenty times at different sites, whereby a distribution line of the maximum inclusion was obtained. On the basis of the obtained distribution line, the maximum flaw area, $(area_{max, m})^{1/2}$ which was present in the minimum broken-flaw area ($\pi r^2 = \pi \times 6^2 = 28.3$ mm$^2$, r represents radius) of the fatigue test piece was obtained. The result is shown in Table 3.

Table 3

Results of Inclusion Test

| Type of Steel | Heat | Symbol representing Heating Treatment | Type of Inclusion | $\sqrt{area}_{max}$ (μm) 28.3mm$^2$ | EPMA Analysis | |
|---|---|---|---|---|---|---|
| | | | | | Component | Type of inclusion |
| SUP12 | A | QT | D D(Ti) | 15 | Al Ti>Zr>N | Al$_2$O$_3$ TiN·ZrN |
| SUP12 | B2 | | D D(Ti) | 18 | Al>Ca>Mg>Si Ti>V>Zr, N | Al$_2$O$_3$·CaO·MgO·SiO$_2$ TiN·VN·ZrN |
| | C1 | | D D(Ti) | 12 | Si>Ca>Al>Mn Ti>V>Zr | SiO$_1$·CaO·Al$_2$O$_3$·MnO·MgO TiN·VN·ZrN |
| SWOSC-V | E2 | | D | 11 | Si>Ca>Mg>Al>Mn>Zr | SiO$_1$·CaO·MgO·Al$_2$O$_3$·ZrO$_3$ |
| | F | | D | 11 | Si□Ca>Zr>Al | SiO$_2$·CaO·MgO·Al$_2$O$_3$·ZrO$_3$ |
| SUP12 | D1 | AF | D | 6 | Al>Mg | Al$_2$O$_3$·MgO |
| | | | | 5 | | |
| | | | | 14 | | |

From Table 3, it is understood that the inclusion which exhibits the $(area_{max, m})^{1/2}$ value of 15 μm is the $Al_2O_3$-

based inclusion, in the SUP12 Steel A Heat. However, from Table 3, it is also understood that, in the other four types of SUP12 steel (B2, C1, D1 Heat) and two types of SWOSC-V steel (E2, F Heat), the inclusions are the $Al_2O_3$-based composite inclusion or the $SiO_2$-based composite inclusion. The latter results indicate that the advanced inclusion-softening control has been carried out therein.

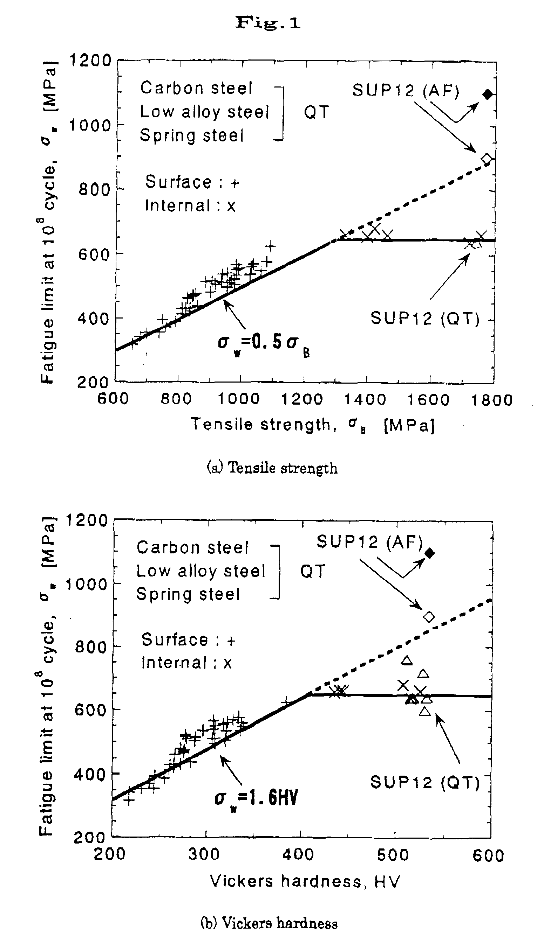

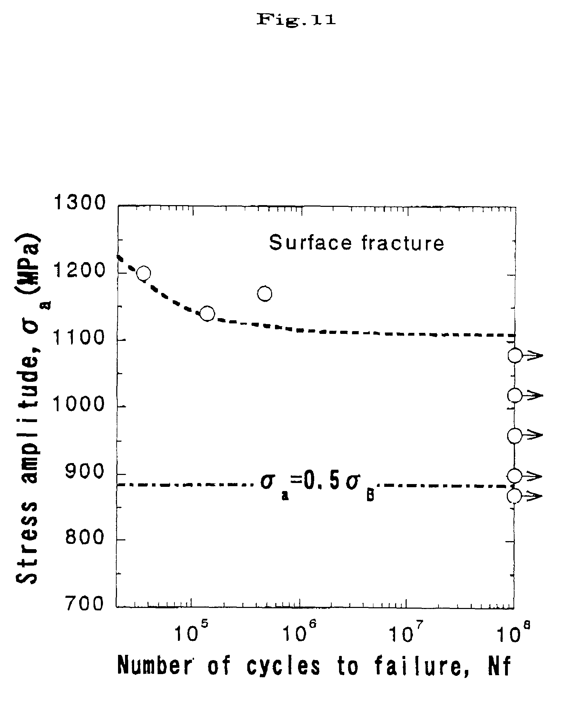

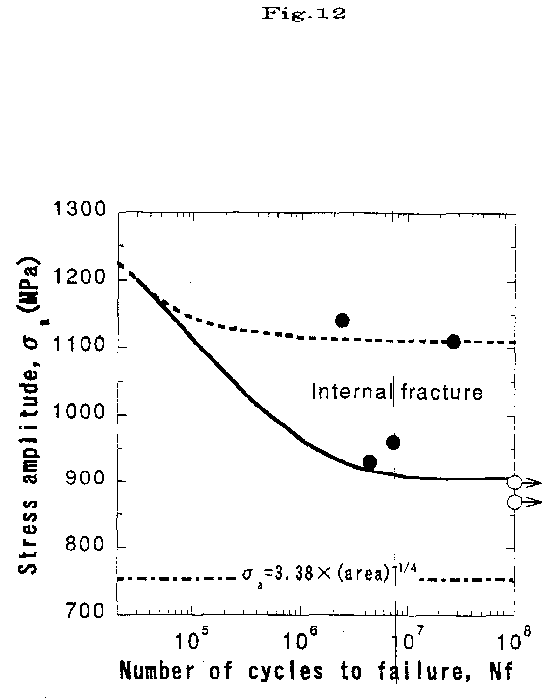

The relationship between the tensile strength $\sigma_B$ of high tensile strength steel of 1200-MPa-exceeding grade (the present invention is directed to the steel of this type) and the $10^8$-cydes fatigue limit is shown in Fig. 1(a). The relationship between the Vickers hardness Hv and the $10^8$-cydes fatigue limit, that is the fatigue limit at $10^8$ cydes is shown in Fig. 1(b). These figures show the surface fracture symbol ♦ and the internal fracture symbol ◊ of the AF material of SUP12 steel, as well as the internal fracture symbol Δ of the QT material of SUP12 steel. In Figs. 1(a) and 1(b), the $10^8$-times fatigue limit obtained from Fig. 11 and Fig. 12 described below is indicated by using the symbol ♦ and the symbol ◊. As the flaw area of the internal fracture (the symbol ◊), the average value 20 μm of the flaw area of the inclusion, which does not have ODA and observed in the broken surface, was used. In addition, in Figs. 1(a) and 1(b), the surface fracture was indicated by the symbol + and the internal fracture was indicated by the symbol × for low tensile strength steel (carbon steel, low alloy steel, spring steel), as well. The result of the internal fracture of the low tensile strength steel (indicated by the symbol × in the figures) shows that the fracture origin is the $Al_2O_3$-based inclusion. In the low tensile strength steel, the relationship: $\sigma_w = 0.5\sigma_B$ is observed. However, in the high tensile strength steel including the modified ausform material (the AF material) of SUP12 steel and the conventionally-quenched-and-tempered material (the QT material) of SUP12 steel, no correlation between the tensile strength and the fatigue limit is observed.

From Figs. 1(a) and 1(b), it is understood that, in the AF material, $\sigma_w$ exceeds $0.5\sigma_B$ or $\sigma_w$ exceeds 1.6 Hv even if the internal fracture has occurred therein. Accordingly, it can be concluded that steel having excellently high fatigue strength can be designed according to the method of evaluating a high fatigue strength material of the present invention.

B Method of evaluating a high fatigue strength material in which the internal fracture origin is considered

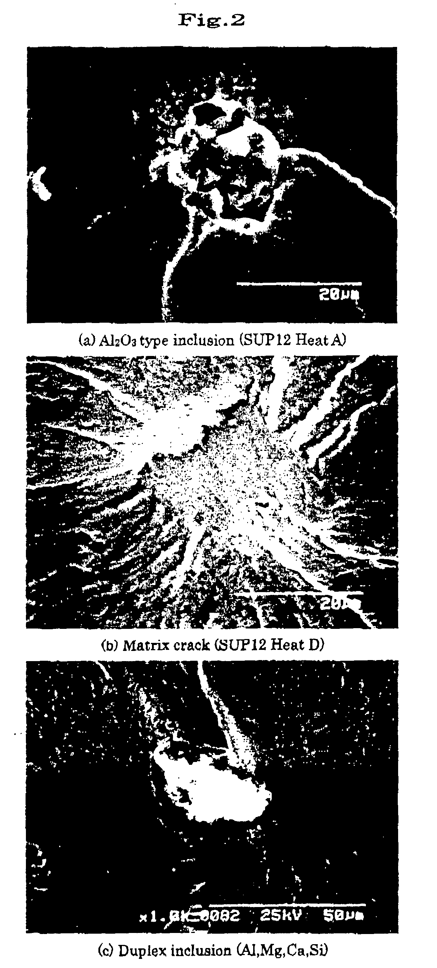

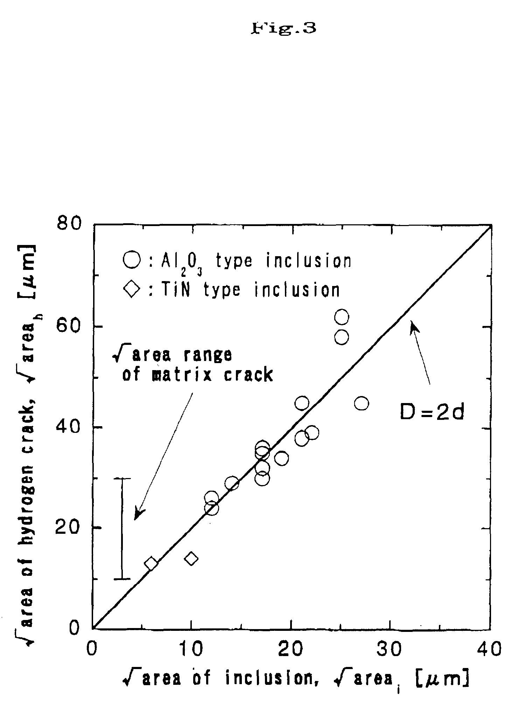

In recent years, it has been known that the optically dark are (ODA) is formed around inclusion prior to generation of ordinary fatigue cracks, which ODA is observed in a SEM (scanning electron microscope) photograph of the inclusion and the vicinities thereof (refer to Fig. 2(a) which is a SEM photograph of the internal fracture origin and the vicinities thereof), and such an ODA increases the effective flaw area of the inclusion. Therefore, the present example considered this point, as well. Specifically, as a result of analyzing the high tensile strength steel on which the fatigue test had been carried out, it was discovered that there is a correlation, as shown in Fig. 3, between the flaw area of the ODA and the flaw area of the inclusion (i.e., D = 2d).

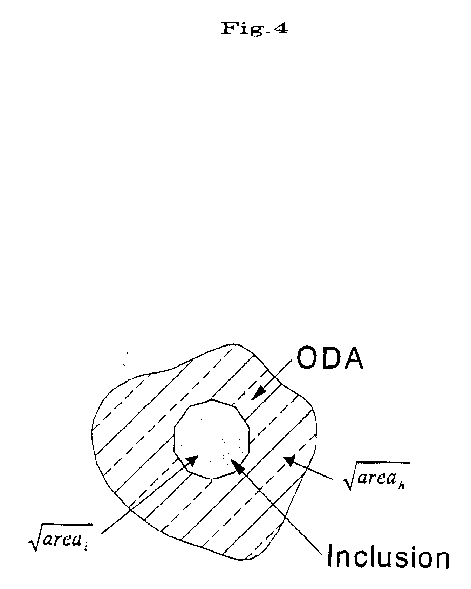

Here, "D" is expressed by the vertical axis and represents the whole flaw area, $(area_h)^{1/2}$, that includes the flaw area of the ODA resulted from the inclusion-originated internal fracture, as well. On the other hand, "d" is expressed by the horizontal axis and represents the flaw area, $(area_i)^{1/2}$, that is of the case in which the fracture origin is located inside the steel (i.e., the case in which there is no influence of ODA) and constituted of only inclusion. This state is schematically shown in Fig. 4.

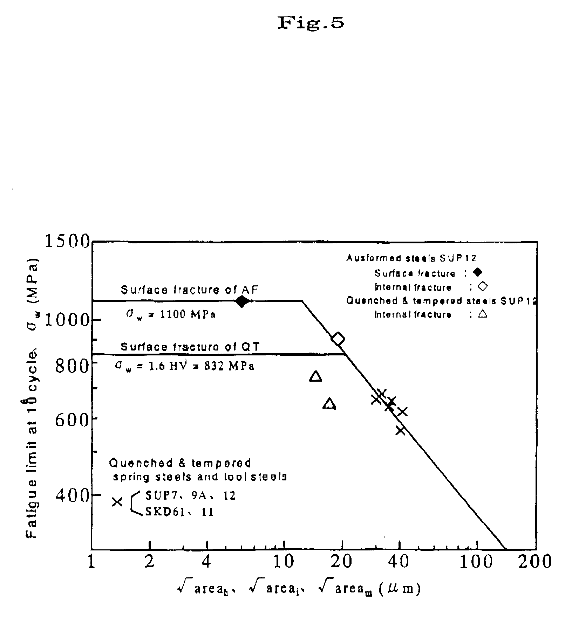

On the basis of the aforementioned findings, the relationship between the fatigue limit $\sigma_w$ and the flaw area, $(area)^{1/2}$, has been arranged as shown in Fig. 5. In Fig. 5, the $10^8$-cydes fatigue limit obtained from Fig. 11 and Fig. 12 described below is indicated by the symbol ♦ and the symbol ◊. The flaw area of the surface fracture (the symbol ♦) is based on the assumption that the maximum inhomogeneous structure flaw area, $(area_{max,m})^{1/2}$, of the D1 Heat AF material of Table 3 can take the value of 5 μm. Here, as the flaw area, $(area)^{1/2}$, of the horizontal axis, the whole flaw area, $(area_h)^{1/2}$, that includes the ODA in the case in which the inclusion-originated internal fracture occurs; the flaw area, $(area_i)^{1/2}$, that is constituted of only the inclusion in the case in which the flaw area of inclusion is not under control; and the flaw area, $(area_{max,m})^{1/2}$, in the case in which the structure crack-originated internal fracture occurs, are each employed. The line (A) in Fig. 5, which represents the aforementioned correlation, can be expressed as follows.

<div align="center">

Equation (5)

</div>

$$\sigma_w = \frac{\Delta K_{th}}{0.666 \cdot \sqrt{\sqrt{\pi} \cdot \sqrt{area}}} = 3.38(area)^{-1/4} \qquad \Box 5 \Box$$

Here, the line (A) represents the correlation when $\Delta K_{th}$ is 3 MPam$^{1/2}$. The line (B) is obtained when the fatigue limit of the surface fracture $\sigma_w$ satisfies either $\sigma_w \geq 0.5\sigma_B$ or $\sigma_w \geq 1.6$Hv. From the intersect of the line (A) and the line (B), the maximum flaw area, $(area)^{1/2}_{max}$, can be expressed as follows.

Equation (6)

$$( (\sqrt{area})_{max} = 45.8/\sigma_B{}^2 \ or \ (\sqrt{area})_{max} \ 4.47/Hv^2 \qquad \square 6 \square$$

From the equation (6), it is understood that, in the present invention, by setting the flaw area at a value which is no larger than the lower limit, the fatigue limit of the resulting high tensile strength steel becomes no less than 0.5 times as much as the tensile strength or no less than 1.6 times as much as the Vickers hardness.

C The case in which the inclusion flaw area is not under control

On the other hand, regarding the inclusion-originated internal fracture, if it is assumed that there is hardly any influence of ODA around the inclusion therein, the flaw area becomes, as shown in Fig. 2, the flaw area $(area_i)^{1/2}$ which is constituted of only the inclusion and is a half of the flaw area that includes the ODA, $(area_h)^{1/2}$. As a result, high tensile strength steel whose tensile strength is substantially the same as that of the conventional high tensile strength steel but whose fatigue limit $\sigma_w$ satisfies the equation $\sigma_w = 3.38 \ (area_i)^{-1/4}$ can be obtained.

Example 2

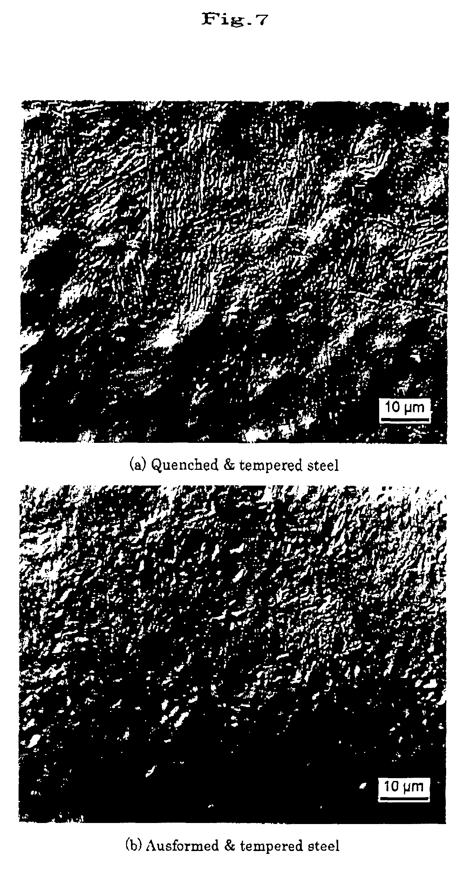

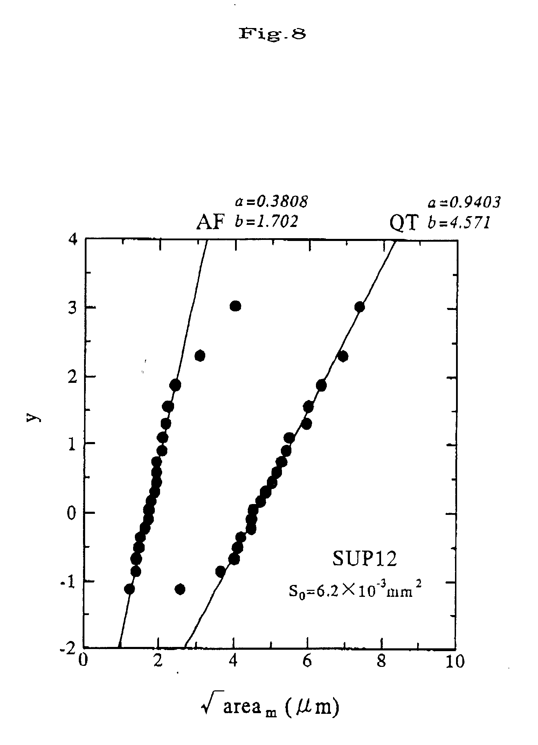

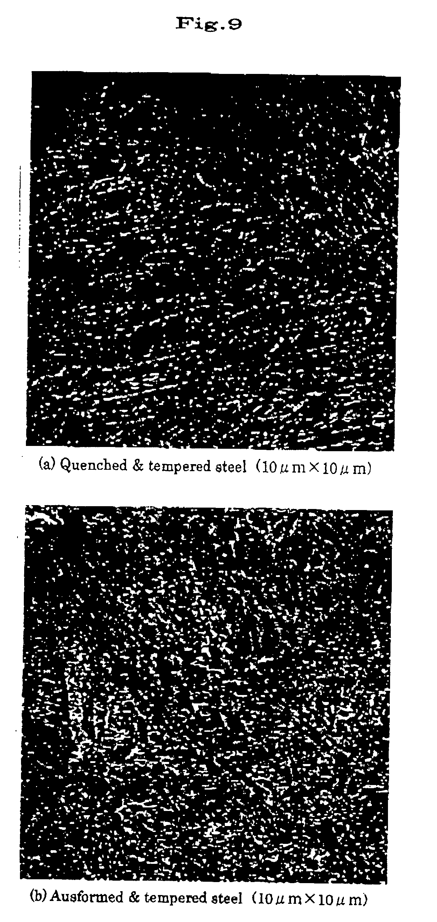

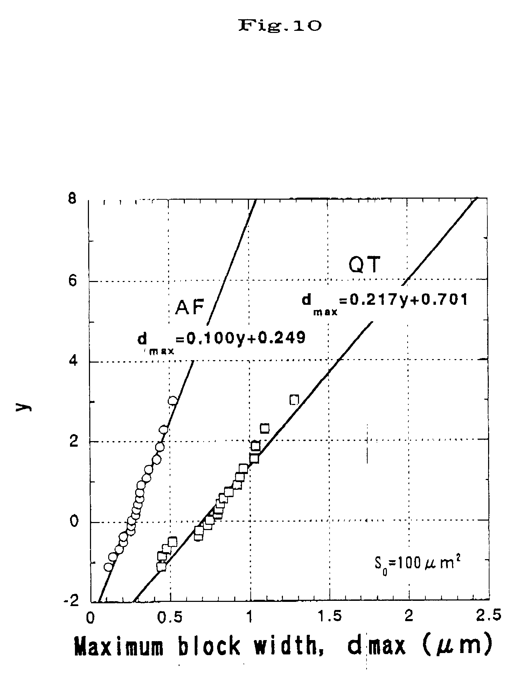

Next, for the SUP12 steel in which the structure thereof had been made homogeneous and the grains thereof had been made minute, high tensile strength steel was designed by applying the aforementioned method of evaluating a high fatigue strength material of the present invention.

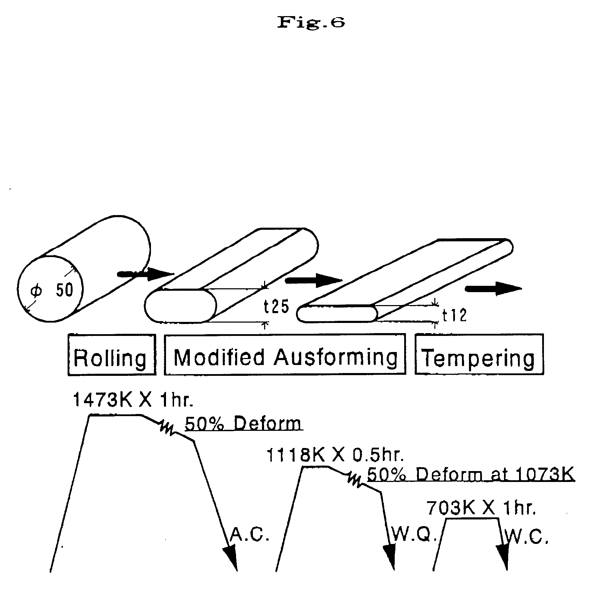

Fig. 6 illustrates the process of the heating treatment, which is applied to SUP12 steel so that the structure of the steel is made homogeneous and the grains of the steel is made minute. Specifically, a round rod having diameter of 50 mm was first held in an electric oven at 1200 °C for 1 hour as the pre-treatment. Thereafter, the rod was rolled so as to have plate thickness of 25 mm and then cooled in the air. As the modified ausforming process, the rolled plate was held in the electric oven at 845 °C for 30 minutes, cooled in the air to 800 °C, rolled so as to have plate thickness of 12 mm by two passes, and then cooled again by water. As the tempering process, the steel was held in a salt bath at 430 °C for 1 hour and then cooled by water. The Vickers hardness of the steel was 534.
Examples of the structures of the modified ausform material (the AF material) and the conventionally-quenched-and-tempered material (the QT material) are shown in the photographs of Fig. 7. Each structure includes inhomogeneous structure. The results of statistically analyzing the maximum-minimum range of the flaw area, $(area)^{1/2}$, of the inhomogeneous structure are shown in Fig. 8. The distribution of the maximum-minimum range of the maximum inhomogeneous structure flaw area, $(area_{max, m})^{1/2}$ of the AF material was found within the range defined by the distribution line of the QT material: $(area_{max, m})^{1/2} = 0.9403y + 4.571$ and the line: $(area_{max, m})^{1/2} = 0$.
Examples of the structure of the tempered martensite of the AF material and the QT material are shown in Fig. 9. When the representative flaw area of the structure is regarded as "block width", the result of statistically analyzing the maximum- minimum range thereof can be plotted as in Fig. 10. The distribution of the maximum-minimum range of the maximum block width $d_{max}$ of the AF material was found within the range defined by the distribution line of the QT material: $d_{max} = 0$ and the line: $d_{max} = 0.217y + 0.701$.
The results, in the case of surface fracture, of the fatigue test of the AF material characteristically having such a structure as described above are shown in Fig. 11. Fig. 11 shows the relationship between the fracture repetition number Nf and the stress magnitude $\sigma a$. In Fig. 11, when the flaw area, $(area)^{1/2}$, is no larger than $45.8/\sigma_B{}^2$, the condition that $\sigma_w \geq 0.5\sigma_B$, which corresponds to the first aspect of the present invention, is satisfied.
The results, in the case of internal fracture, of the fatigue test of the AF material are shown in Fig. 12. Fig. 12 shows the relationship between the fracture repetition number Nf and the stress magnitude $\sigma a$. In Fig. 12, when the flaw area, $(area)^{1/2}$, is larger than $45.8/\sigma_B{}^2$, internal fracture is generated by the inclusion whose flaw area, $(area_i)^{1/2}$, average is approximately 20 μm. However, as there exists no ODA around the inclusion, the condition that $\sigma_w \geq 3.38$ $(area_i)^{-1/4}$, which corresponds to the second aspect of the present invention, is satisfied.

Example 3

The SUP12 steel produced by the conventional heating treatment was heated to 300 °C in high vacuum of $2 \times 10^{-6}$ Pa or higher. The Vickers hardness of the steel was 518. The flaw area, $(area)^{1/2}$, was no larger than $4.8/\sigma_B{}^2$, and the condition that $\sigma_w \geq 1.6$ Hv was satisfied.
As described above in detail, the present invention can provide a method of designing high fatigue strength material in high tensile strength steel, in which method the relationship between the flaw dimension (area) of ODA and the fatigue strength is considered. The present invention can also provide a high fatigue strength material itself.

LEGEND TO THE FIGURES

Fig. 1

(a): Tensile Strength
$10^8$-times Fatigue Strength, $\sigma_w$(MPa)
Tensile Strength, $\sigma_B$ (MPa)
SUP12 steel AF material
Surface fracture: ♦
Internal fracture: ◊
SUP12 steel QT material
Internal fracture: Δ
Conventional carbon steel, low alloy steel, spring steel Surface fracture: +
Internal fracture: ×
(b): Vickers Hardness
$10^8$-times Fatigue Strength, $\sigma_w$(MPa)
Vickers hardness, HV
SUP12 steel AF material
Surface fracture: ♦
Internal fracture: ◊
SUP12 steel QT material
Internal fracture: Δ
Conventional carbon steel, low alloy steel, spring steel Surface fracture: +
Internal fracture: ×

Fig. 2

(a): Fracture origin of Heat A of SUP12 steel --- $Al_2O_3$- based inclusion
(b): Fracture origin of Heat D1 of SUP12 steel --- Structure cracking
(c): Fracture origin-composite inclusion (Al, Mg, Ca, Si)

Fig. 3
$(Area)^{1/2}$, $(Area_h)^{1/2}$ of Hydrogen-originated cracking area (μm)
$(Area)^{1/2}$, $(Area_i)^{1/2}$ of Inclusion (μm)
Conventional spring steel
$(Area)^{1/2}$ range of structure cracking

Fig. 4
Inclusion

Fig. 5
$10^8$-times Fatigue Strength, $\sigma_w$(MPa)
$(Area)^{1/2}$, $(Area_h)^{1/2}$, $(Area_i)^{1/2}$, $(Area_m)^{1/2}$ (μm)
AF surface fracture
QT surface fracture
Line (A)
Line (B)
Axial-direction load applying test and rotational bending test of conventional spring steel and tool steel
SUP12 steel AF material
Surface fracture: ♦
Internal fracture: ◊
SUP12 steel QT material
Internal fracture: Δ

Fig. 6
Heating Treatment Process

Fig. 7

(a) Conventionally-heat-processed material (QT material)
(b) Ausform material (AF material)

Fig. 9

(a): Observation of Block Structure (AFM)
Conventionally-heat-processed material (QT material)
[Observed range: 10 µm $\times$ 10 µm]
(b): Observation of Block Structure (AFM)
Ausform material (AF material) [Observed range: 10 µm $\times$ 10 µm]

Fig. 10
Standardizing Parameter, y
Maximum Block Width, dmax (µm)
Ausform material
Conventionally-heat-processed material

Fig. 11
Result of Fatigue Test of AF Material (Inclusion are under control)
Stress Magnitude, $\sigma_a$(MPa)
Fracture Repetition Number, Nf
Surface Fracture

Fig. 12
Result of Fatigue Test of AF Material (Inclusion are not under control)
Stress Magnitude, $\sigma_a$(MPa)
Fracture Repetition Number, Nf
Internal Fracture

**Claims**

1. A method of evaluating a high fatigue strength material in high tensile strength steel, comprising the steps of:

    obtaining the values of tensile strength $\sigma_B$ (unit thereof is MPa) and Vickers hardness Hv of the steel; and estimating, in designing the high fatigue strength material, that the fatigue limit $\sigma_w$ (unit thereof is MPa) of the steel satisfies either $\sigma_w \geq 0.5\sigma_B$ or $\sigma_w \geq 1.6$Hv, when the fracture origin is located only at a surface of the steel and a square root of the flaw area, (area)$^{1/2}$ (unit thereof is m), contained in the steel is no larger than $45.8/\sigma_B^2$ or $4.47/\text{Hv}^2$.

2. A method of evaluating a high fatigue strength material in high tensile strength steel, comprising the steps of:

    obtaining the values of tensile strength $\sigma_B$ (unit thereof is MPa) and Vickers hardness Hv of the steel;
    measuring the flaw area of inclusion, when the fracture origin is located inside the steel; and
    estimating, in designing the high fatigue strength material, that the fatigue limit $\sigma_w$ (unit thereof is MPa) of the steel satisfies a condition that $\sigma_w \geq 3.38$ (area$_i$)$^{-1/4}$

3. A method of evaluating a high tensile strength structure, which method enabling the evaluation of the high fatigue strength material according to claim 1 or 2, which method comprising the steps of:

    measuring the maximum inhomogeneous structure flaw area, (area$_{max,m}$), when the structure thereof has been made homogeneous (i.e., the size of the inhomogeneous structure has been made small) or the structure thereof has been made minute (i.e., the block width thereof has been reduced);
    setting the distribution of the maximum-minimum range of the maximum inhomogeneous structure flaw area, (area$_{max, m}$)$^{1/2}$, (unit thereof is µm) within the range defined by the line: (area$_{max, m}$)$^{1/2}$ = 0 and the line: (area$_{max, m}$)$^{1/2}$ = 0.9403y + 4.571 (the test standard area $S_0 = 6.2 \times 10^{-9}$m$^2$); and

setting the distribution of the maximum-minimum range of the maximum block width $d_{max}$ (unit thereof is μm) within the range defined by the line: $d_{max} = 0$ and the line: $d_{max} = 0.217y + 0.701$ (y is a standardizing parameter, the test standard area being $1 \times 10^{-10} m^2$).

4. A method of producing a high fatigue strength material, which method comprising producing steel according to the method of evaluating a high fatigue strength material in high tensile strength steel of claim 1 or 2, and according to a method of enabling evaluation of a high tensile strength structure, which high tensile strength structure evaluating method comprising the steps of:

> measuring the maximum inhomogeneous structure flaw area, $(area_{max,m})$, when the structure thereof has been made homogeneous (i.e., the size of the inhomogeneous structure has been made small) or the structure thereof has been made minute (i.e., the block width thereof has been reduced);
> setting the distribution of the maximum-minimum range of the maximum inhomogeneous structure flaw area, $(area_{max, m})^{1/2}$, (unit thereof is μm) within the range defined by the line: $(area_{max, m})^{1/2} = 0$ and the line: $(area_{max, m})^{1/2} = 0.9403y + 4.571$; and
> setting the distribution of the maximum-minimum range of the maximum block width $d_{max}$ (unit thereof is μm) within the range defined by the line: $d_{max} = 0$ and the line: $d_{max} = 0.217y + 0.701$ (y is a standardizing parameter).

5. A method of producing a high fatigue strength material, which method including producing steel according to the method of claim 1, by subjecting steel to a heating process for tempering the steel in high vacuum of $2 \times 10^{-6}$ Pa or higher.

6. A high fatigue strength material, produced by the method of evaluating a high fatigue strength material in high tensile strength steel according to claim 1 or 2, and also by a method of enabling evaluation of a high tensile strength structure, which high tensile strength structure evaluating method comprising the steps of:

> measuring the maximum inhomogeneous structure flaw area, $(area_{max,m})$, when the structure thereof has been made homogeneous (i.e., the size of the inhomogeneous structure has been made small) or the structure thereof has been made minute (i.e., the block width thereof has been reduced);
> setting the distribution of the maximum-minimum range of the maximum inhomogeneous structure flaw area, $(area_{max, m})^{1/2}$, (unit thereof is μm) within the range defined by the line: $(area_{max, m})^{1/2} = 0$ and the line: $(area_{max, m})^{1/2} = 0.9403y + 4.571$; and
> setting the distribution of the maximum-minimum range of the maximum block width $d_{max}$ (unit thereof is μm) within the range defined by the line: $d_{max} = 0$ and the line: $d_{max} = 0.217y + 0.701$ (y is a standardizing parameter).

## Fig.1

(a) Tensile strength

(b) Vickers hardness

Fig.2

(a) Al₂O₃ type inclusion (SUP12 Heat A)

(b) Matrix crack (SUP12 Heat D)

(c) Duplex inclusion (Al,Mg,Ca,Si)

Fig.3

Fig.4

ODA

$\sqrt{area_h}$

$\sqrt{area_i}$

Inclusion

Fig.5

## Fig.6

Rolling | Modified Ausforming | Tempering

φ 50
t25
t12

1473K X 1hr.
50% Deform
1118K X 0.5hr.
50% Deform at 1073K
703K X 1hr.
A.C.
W.Q.
W.C.

Fig.7

(a) Quenched & tempered steel

(b) Ausformed & tempered steel

Fig.8

## Fig.9

(a) Quenched & tempered steel $(10 \mu m \times 10 \mu m)$

(b) Ausformed & tempered steel $(10 \mu m \times 10 \mu m)$

Fig.10

Maximum block width, d max ($\mu$m)

Fig.11

Fig.12